# EUROPEAN PATENT APPLICATION

(11) **EP 4 640 166 A1**
(43) Date of publication of application: **29.10.2025**
(21) Application number: 23905969.4
(22) Date of filing: 19.12.2023
(51) Int. Cl.: A61B 17/04

(54) **ENDOSCOPIC SUTURING INSTRUMENT**

(30) Priority: 22.12.2022 CN 202211652044
(71) Applicant: B. J. Zh. F. Panther Medical Equipment Co., Ltd., Beijing 102200 (CN)
(72) Inventor: FAN, Hongli, Beijing 102200 (CN)
(74) Representative: Huang, Liwei
(86) International application number: PCT/CN2023/140008
(87) International publication number: WO 2024/131806

(57) **Abstract**

Disclosed is an endoscopic suture device, comprising: a working end, and a pressing-piece driving component, wherein the working end comprises a pressing-piece, the pressing-piece is used for limiting the detachment of a suture needle from a track, the pressing-piece driving component can relieve the restraint of the pressing-piece on the suture needle, and the pressing-piece driving component comprises: a rotating shaft sleeve, a rotating inner shaft, a first limiting component, a steel wire shaft and a steel wire rope; the rotating inner shaft is provided in the rotating shaft sleeve, and the first limiting member is used for limiting the rotating inner shaft so that the rotating inner shaft cannot move axially relative to the rotating shaft sleeve but can rotate relative to the rotating shaft sleeve; One end of the steel wire shaft is fixedly connected to the pressing-piece, and the other end is fixedly connected to the rotating inner shaft; and one end of the steel wire rope is fixedly connected to the rotating shaft sleeve. The working end of the endoscopic suture device of the present invention can achieve 360 degree rotation, and does not cause an instrument fault due to the winding of a steel wire rope, thereby better meeting the requirements of a surgery.

## Description

### Technical Field

The present invention relates to the field of medical devices, and in particular, to an endoscopic suture device.

### Background

Microscopic surgery is a newly developed minimally invasive method, and is an inevitable trend of future surgical method development. However, due to the limitation of the space inside the abdominal cavity or the thoracic cavity, the minimally invasive surgical instrument is necessarily small and long, so that an accurate operation can be achieved.

A relatively high level of operating difficulty in minimally invasive abdominal surgery is undoubtedly suturing. The endoscopic suture device plays a very critical role in surgery.

After suturing of the device is completed, the suture needle needs to be thrown away. In the prior art, the working end presser is directly connected via a steel wire rope, and when the suture needle needs to be thrown away, an external operation handle is pulled, so that the working end presser can be pulled away from the suture needle, the restriction on the suture needle is lifted, and the suture needle is dropped. By means of directly connecting a steel wire rope to a pressing-piece at a working end, the existence of the steel wire rope limits the free rotation of the working end, so that the steel wire rope cannot realize 360 degrees rotation, and cannot completely satisfy the requirements of surgery. In addition, during the working process, the working end rotates, but the external operating handle is not rotated in the same direction as the working end by means of pulling outside the body, so that the steel wire rope is wound inside, thereby damaging the instrument.

### Summary

The present invention solves the technical problems in the prior art that the working end of an endoscopic suture device cannot rotate freely and a steel wire rope is wound around a damage instrument.

In order to solve the described technical problem, the present invention provides the following technical solutions:
An endoscopic suture device, comprising: a working end, a pressing-piece driving component, wherein the working end comprises a pressing-piece, the pressing-piece is used for restraining a suture needle from being separated from a track, and the pressing-piece driving component is able to release the restraint of the pressing-piece on the suture needle, the pressing-piece driving component comprises: a rotating shaft sleeve, a rotating inner shaft, a first limiting component, a steel wire shaft, a steel wire rope; the rotating inner shaft is arranged in the rotating shaft sleeve, the first limiting component is used for limiting the rotating inner shaft so that the rotating inner shaft is not able to move axially relative to the rotating shaft sleeve but is able to rotate relative to the rotating shaft sleeve; one end of the steel wire shaft is fixedly connected to the pressing-piece, and the other end is fixedly connected to the rotating inner shaft; one end of the steel wire rope is fixedly connected to the rotating shaft sleeve.

Further, at least one compression spring is further provided at the working end, a compression spring hole is provided on the pressing-piece, and the compression spring is fitted into the compression spring hole to apply compression to the pressing-piece so as to restrain the suture needle from departing from the running track; the pressing-piece is provided with a steel shaft hole for inserting one end of the steel shaft and performing a fixed connection, and the rotating inner shaft is provided with a circular hole for inserting the other end of the steel shaft and performing a fixed connection; the rotating inner shaft is provided with a connection hole for fitting into one end of the steel wire rope and fixedly connecting.

Further, the first limiting member comprises a U-shaped annular groove and a spherical steel ball, wherein the U-shaped annular groove is provided on an outer side wall of the rotating inner shaft, and the rotating shaft sleeve is provided with a plurality of steel ball holes in a radial direction, a spherical steel ball is mounted in each steel ball hole, and the spherical steel ball has a first protruding portion protruding from the inner surface of the rotating shaft sleeve, the first protruding portion is correspondingly provided in the U-shaped annular groove, and the height of the first protruding portion is smaller than the depth of the U-shaped annular groove.

In the present invention, the first limiting member limits the axial movement of the rotating inner shaft relative to the rotating shaft sleeve, so that the rotating inner shaft can only rotate relative to the rotating shaft sleeve. Since the rotating inner shaft cannot axially move relative to the rotating shaft sleeve, if the rotating shaft sleeve moves axially, the rotating inner shaft can be driven to axially move, and at the same time, the rotating inner shaft can rotate 360 degrees relative to the rotating shaft sleeve. In specific operation engineering, a working end can rotate 360 degrees, so that the requirements of a surgery can be satisfied. Furthermore, during the rotation of the working end, only the steel wire shaft rotates along with the rotating shaft sleeve, and the steel wire rope does not need to rotate; therefore, the steel wire rope will not be wound to cause an instrument fault, thereby affecting the safety of the operation.

Further, the endoscopic suture device also comprises a fixed outer tube and a second limiting member, the rotating shaft is sleeved in the fixed outer tube, and the second limiting member is used for limiting the rotating shaft sleeve so that the rotating shaft sleeve is not able to rotate relative to the fixed outer tube but is able to move linearly along the axis relative to the fixed outer tube.

Further, the specific structure of the second limiting member is as follows: the spherical steel ball has a second protruding portion protruding from the outer circumferential surface of the rotating shaft sleeve, and the fixed outer tube is provided with an axial track groove arranged corresponding to the second protruding portion of the spherical steel ball, wherein the height of the second protruding portion is smaller than the depth of the axial track groove;
or, the spherical steel ball is not higher than the surface of the outer side wall of the rotating shaft sleeve after being inserted into the rotating shaft sleeve, the outer side wall of the rotating shaft sleeve is provided with at least one track protrusion, the inner side wall of the fixed outer tube is correspondingly provided with at least one track groove, and the height of the track protrusion is less than the depth of the track groove.

By providing the second limiting member, the present invention limits the rotation of the rotating shaft sleeve relative to the fixed outer tube, but enables the rotating shaft sleeve to move axially in the fixed outer tube under the driving of the steel wire rope, thereby driving the rotating inner shaft to move axially.

Further, the endoscopic suture device also comprises a skeleton, a shifting lever is provided on the skeleton, one end of the steel wire rope is fixed on the shifting lever; when the shifting lever is shifted, the steel wire rope is pulled to move.

Further, the skeleton is provided with a first pin hole and a second pin hole respectively used for inserting the first pin shaft and the second pin shaft; one end of the steel wire rope passes through a steel wire hole provided on the shifting lever and is fixed; the shifting lever is provided with a second pin shaft hole for passing through a second pin shaft, and the shifting lever can rotate by taking the second pin shaft as an axis; further comprising a steel wire rotating wheel, wherein the steel wire rotating wheel is provided with a central hole for inserting the first pin shaft; and the steel wire rope is embedded into a U-shaped groove provided on the steel wire rotating wheel.

Further, the device further comprises a cushioning component, the cushioning component is disposed between the shift lever and the rotating shaft sleeve, the steel wire rope is broken at the cushioning component into two, and two ends of the cushioning component are connected to the steel wire ropes respectively so as to connect the broken steel wire ropes; the cushioning component comprises a cushioning sleeve, a cushioning compression spring, a cushioning sleeve cover, and a spring compression block.

Further, the cushioning compression spring and the spring compression block are disposed in the cushioning sleeve, one end of the cushioning compression spring abuts the inner side of the bottom of the cushioning sleeve, and the other end abuts the spring compression block; one steel wire rope passes through the bottom through-hole of the cushioning sleeve tube, a cushioning compression spring and a spring pressing block in sequence and is then fixed; and the other steel wire rope passes through the cover of the cushioning sleeve tube and is then fixed, and the cushioning sleeve tube is fixedly connected to the cover of the cushioning sleeve tube.

Further, the elastic force F_{cushioning compression spring} of the cushioning compressing spring is less than the tensile strength of the steel wire rope and the strength of other relevant parts, and satisfies the following relationship: F_{cushioning compression spring}>2×F_{compression spring}, wherein F_{compression spring} refers to the elastic force of the compression spring at the working end.

After the design of the invention is adopted, the invention has at least the following advantages:
(1) The working end of the endoscopic suture device can rotate 360 degrees, so that the requirement of an operation can be better met.
(2) The endoscopic suture device can not cause the steel wire rope to be wound to cause instrument failure, thereby ensuring the operation safety.
(3) The endoscopic suture device can improve the safety of the operation process of the instrument by arranging the cushioning component, and avoids damaging related parts due to overlarge pulling force.
(4) The invention has the advantages of easy realization of the structure, good effect and low cost.

### Drawings

FIG. 1 is an exploded view of the needle pressing-piece driving component of the endoscopic suture device according to the present invention;
FIG. 2 is a schematic view of a needle clip of the present invention;
FIG. 3 is a schematic cross-sectional view of a needle pressing-piece driving component of the endoscopic suture device according to the present invention;
FIG. 4 is a schematic view of a rotating inner shaft according to an embodiment of the present invention;
FIG. 5 is a schematic view of a rotating inner shaft of another embodiment of the present invention;
FIG. 6 is a schematic view of a fixed outer tube according to an embodiment of the present invention;
FIG. 7 is a schematic view of a stationary outer tube according to another embodiment of the present invention;
FIG. 8 is a schematic view of the operational end piece of the present invention;
FIG. 9 is a schematic view of a driving lever of the present invention;
FIG. 10 is a cross-sectional view of a cushioning member of the present invention;
FIG. 11 is a schematic view of the operational movement of the endoscopic suture device of the present invention.

### [Description of the reference numerals]

1000 - Needle press tab drive component, 1100 - Fixed outer tube, 1101 - Orbital groove A, 1102 - Orbital groove B, 1103 - Orbital groove C, 1104 - Orbital groove D, 1200 - Rotating shaft sleeve, 1201 - Steel ball hole A, 1202 - Steel ball hole B, 1203 - Steel ball hole C, 1204 - Steel ball hole D, 1205 - Connection hole, 1206 - Orbital A 1207-Track B, 1300-Rotating Inner Shaft, 1301-Circular Hole, 1302-U-Shaped Ring Groove, 1400-Spherical Ball 41, 2000-Working End, 2100-Wire Shaft, 2200-Compression Spring, 2300-Compression Needle Sheet, 2301-Spring A Hole, 2302-Spring B Hole, 2303-Wire Hole, 2400-Suture Needle, 3000-Operating End Parts, 3100-Toggle, 3101-Pin Holes, 3102-Wire Holes, 3103-Toggle Knobs, 3200-Wire Runners, 3201 -U Grooves, 3202-Center Holes, 3300-Wire Cord, 3400-Compression Sleeve , 3500-Pins, 3600-Skeleton, 3601-First Pin Holes, 3602-Second Pin Holes, 3603-Support Table, the 4000-cushioning parts, 4100-cushioning casing, 4200-reducing compression spring, 4300-cushioning casing cover, 4400-spring compression block.

### Specific embodiments

The technical solutions in the embodiments of the present invention will be clearly and completely described below in conjunction with the accompanying drawings in the embodiments of the present invention, and it is clear that the described embodiments are only a part of the embodiments of the present invention and not all of the embodiments. Based on the embodiments in the present invention, all other embodiments obtained by persons of ordinary skill in the art without making creative labor are within the scope of protection of the present invention.

In this document, "up", "down" and the like are used only to indicate relative positional relationships between related parts, and not to define the absolute positions of these related parts.

Each of the following parts is defined as a proximal end near the operator and a distal end away from the operator. The proximal and distal expressions are used only to make the description simple and clear and should not be construed as any limitation of the present invention.

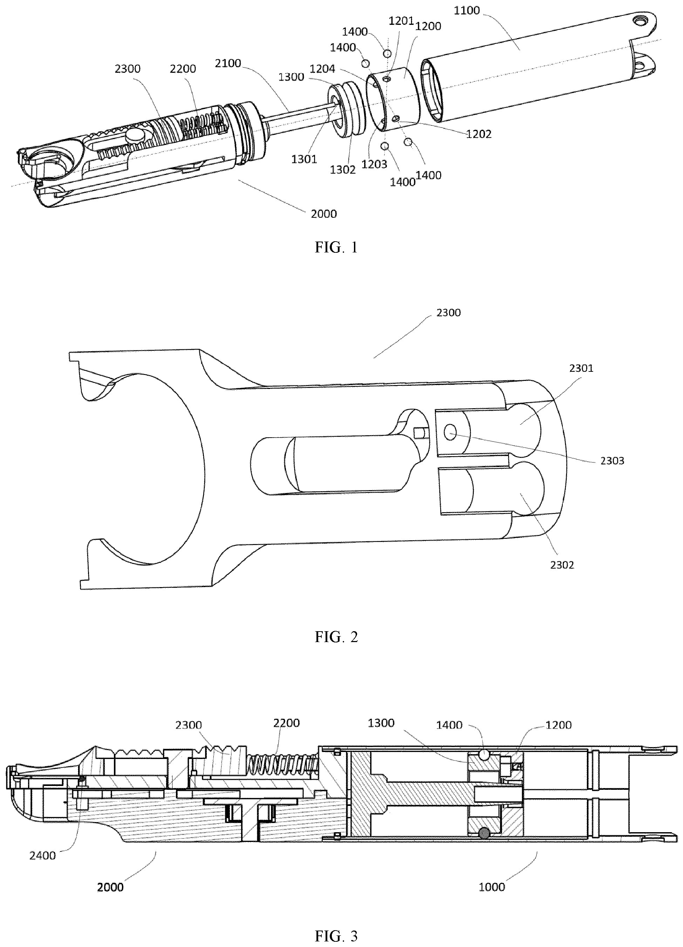

As shown in FIGS. 1, 3 and 11, a luminal suture apparatus comprising: a working end 2000, a compression needle sheet driving part 1000, the working end comprising a compression needle sheet 2300, the compression needle sheet 2000 for restraining a suture needle 2400 from being disengaged from a track; the compression needle sheet driving part 1000 for releasing the restriction of the compression needle sheet 2300 on the suture needle 2400; the compression needle sheet driving part 1000 comprising: a rotating shaft sleeve 1200, rotary inner shaft 1300, first limit part, steel wire shaft 2100, steel wire rope 3300; rotary inner shaft 1200 is provided in rotary shaft sleeve 1300, the first limit part is used to restrict rotary inner shaft 1300 so that rotary inner shaft 1300 cannot move axially relative to rotary shaft sleeve 1300 but can rotate relative to rotary shaft sleeve 1200; steel wire shaft 2100 is fixedly connected with needle piece 2300 at one end and the other end is fixedly connected with needle piece 2300. One end of the steel wire shaft 2100 is fixedly connected to the needle sheet 2300, and the other end is fixedly connected to the inner axis of rotation 1300; one end of the steel wire rope is fixedly connected to the rotating shaft sleeve 1200.

Specifically, the working end 2000 is used to perform a suturing operation, and is provided with a compression needle sheet 2300 and at least one compression spring 2200, which applies compression to the compression needle sheet by the compression springs 2200 thereby restraining the suture needle 2400 from being disengaged from the running track. As shown in FIG. 2, the compression springs are two, and the compression needle sheet 2300 is provided with a spring A hole 2301 and a spring B hole 302, and the two compression springs 2200 are respectively fitted into the spring A hole 2301 and the spring B hole 2302 to exert compression on the compression needle sheet 2300, and the compression needle sheet 2300 presses against the suture needle 2400 for restraining the suture needle 2400 from being disengaged from the running track. The compression needle piece 2300 is provided with a steel wire shaft hole 2303 for threading into one end of the steel wire shaft 2100 and fixedly connecting thereto, and dragging the steel wire shaft 2100 can drive the compression needle piece 2300 to move so as to release the restriction on the suture needle 2400. The rotating inner shaft 1300 is provided with a circular hole 1301 for fitting into the other end of the steel wire shaft 2100 and fixing the connection thereon. The swivel sleeve 1200 is provided with a connection hole 1205 for fitting and securing one end of the wire rope 3300.

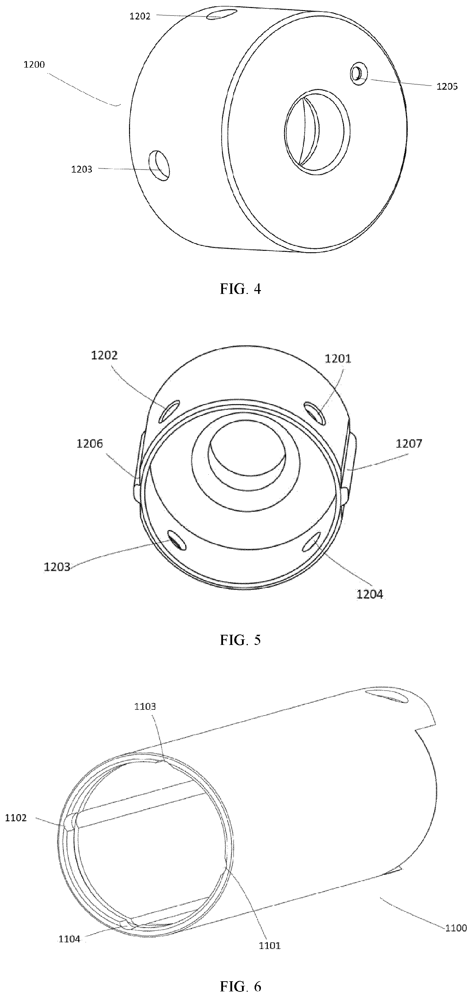

The rotary inner shaft 1300 is provided with a U-shaped annular groove 1302 on the outer side wall, the rotary shaft sleeve 1200 is radially provided with a plurality of steel ball holes, and each of the steel ball holes is provided with a spherical steel ball 1400, the spherical steel ball 1400 has a protruding portion protruding out of the inner surface of the rotary shaft sleeve 1400, the protruding portion of the spherical steel ball 1400 is correspondingly provided in the U-shaped annular groove 1302, wherein the height of the protruding portion is less than the depth of the U-shaped annular groove. depth of the U-shaped annular groove. As shown in FIGS. 4 and 5, the steel ball holes are four, corresponding to steel ball holes A 1201, steel ball holes B 1202, steel ball holes C 1203, and steel ball holes D 1204, each of which is respectively fitted with the spherical steel ball 1400. the U-shaped annular groove, and the spherical steel ball comprise a first limiting member, and the first limiting member restricts an axial movement of the inner rotating shaft 1300 with respect to the rotating bushing 1200 so that the inner rotating shaft 1300 can only move rotationally relative to the rotating shaft sleeve 1200. Since the rotating inner shaft 1300 can realize 360-degree rotation relative to the rotating shaft sleeve 1200, the working end can realize 360-degree rotation during specific operation, so that the needs of surgery can be met.

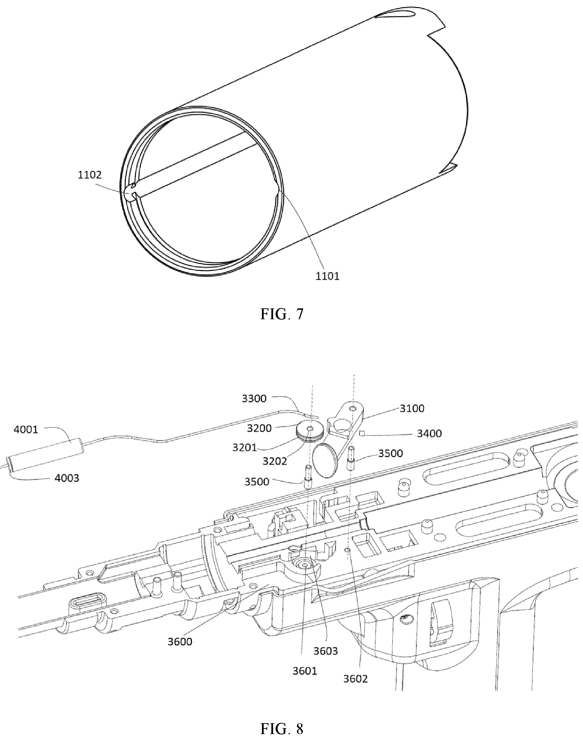

The motorized suture apparatus further includes a fixed outer tube 1100 and a second limiting member, the rotating sleeve 1200 is provided within the fixed outer tube 1100, and the second limiting member is used to limit the rotating sleeve 1200 such that the rotating sleeve is not allowed to rotate with respect to the fixed outer tube 1100 but is allowed to move in a straight line along an axis with respect to the fixed outer tube 100. Specifically, in one embodiment, the spherical steel ball 1400 has a second protruding portion protruding from the outer circumferential surface of the rotating sleeve 1200, as shown in FIG. 3. The fixed outer tube 1100 is provided with axially oriented track slots provided in correspondence with the second protruding portion of the spherical steel balls 1400, wherein the height of the second protruding portion is less than the depth of the track slots, and the number of track slots corresponds to the number of spherical steel balls; if the number of spherical steel balls is four, the track slots are also four, and as shown in FIG. 6, the four spherical steel balls correspond to the track slots A 1101, the 1 track slots B 1102, track groove C 1103, and track groove D 1104. In another embodiment, as shown in FIGS. 5 and 7, the spherical steel ball 1400 is set so that it is not higher than the circumferential surface of the rotary bushing 1200 after it is loaded into the rotary bushing, and the rotary bushing 1200 is provided with at least one track protrusion on the outer wall, and at least one track groove is provided correspondingly on the inner wall of the stationary outer tube, wherein the height of the track protrusion is smaller than the depth of the track groove; preferably, the track projections are two, respectively: track projection A 1206, track projection B 1207; correspondingly, the fixed outer tube 1100 is provided with two track grooves, respectively: track groove A, track groove B. In addition, the number of track grooves is not limited to two, but may be more than two, preferably, the track grooves are uniformly distributed along the inner circumference of the fixed outer tube 1100. The number and distribution position of the track projections of the rotating shaft sleeve 1200 are adapted to the track slots.

The luminal suture apparatus further includes a skeleton 3600, the skeleton 3600 is provided with a toggle 3100, and one end of the wire rope 3300 is fixed to the toggle 3100, so that when the toggle is toggled, the wire rope can be pulled to move.

As shown in FIG. 8, the skeleton 3600 is provided with a first pin hole 3601 and a second pin hole 3602 for inserting the first pin and the second pin, respectively. The wire rope 3300 is fixed after one end is threaded into the wire hole 3102 provided in the toggle bar 3100, and preferably, the wire hole 3102 is fitted with a crimp sleeve 3400 to restrain the wire rope 3300 from coming out. The toggle bar 3100 is provided with a pin hole 3101 for threading a second pin, and the toggle bar 3100 can be rotated on the axis of the second pin. The wire rope 3300 is embedded in a U-shaped groove 3201 provided in the wire runner 3200, and the wire runner 3200 is provided with a center hole 3202 for threading the first pin.

The skeleton 3600 is also provided with a support table 3603 to avoid the wire rope 3300 from being dislodged from the U-shaped groove 3201 provided in the wire runner 3200 during movement.

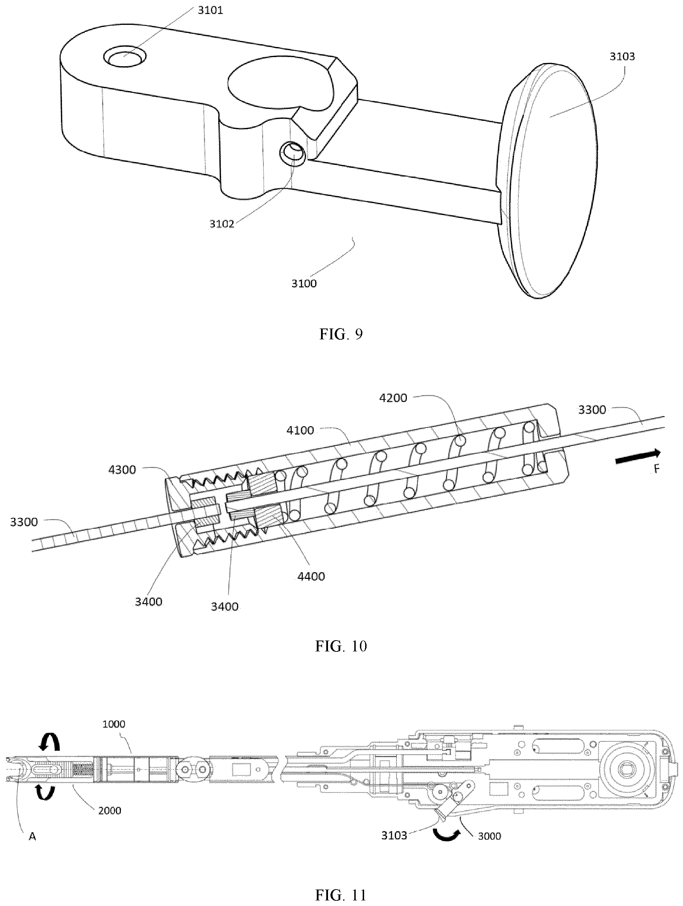

As shown in FIG. 10, the cushioning part 4000 is provided between the toggle 3100 and the rotating sleeve 1200, which is used to improve the safety of the operation process of the apparatus and to avoid damage to the relevant parts due to the excessive tension, and the wire rope 3300 is disconnected and divided into two at the cushioning part 4000, and the ends of the cushioning part are connected to the wire rope 3300 so that the disconnected wire ropes are connected; the cushioning part 4000 includes cushioning casing 4100, cushioning compression spring 4200, cushioning casing cover 4300, spring compression block 4400. cushioning compression spring 4200 and spring compression block 4400 are set in cushioning casing 4100, one end of the compression spring against the bottom inside of the cushioning casing, and one end of the compression spring against the spring compression block; a steel wire rope 3300 passes through the bottom through hole of the cushioning casing 4100, cushioning compression spring 4200 in turn, spring compression block 4400 and then secured; another wire rope passes through the cushioning casing cover 4300 and then secured, and the cushioning casing 4100 is fixedly connected to the cushioning casing cover 4300; preferably, the ends of the two wire ropes are secured by press-fitting through the crimp casing 3400, and the cushioning casing and the cushioning casing cover are connected by threads.

The elastic force of F_{cushioning compression spring} is less than the tensile strength of the steel wire rope and the strength of other relevant parts, and satisfies the following relationship: F_{cushioning compression spring}>2×F_{compression spring}, wherein F_{compression spring} refers to the elastic force of the compression spring at the working end. The above elasticity is set so that both the toggle 3100 can be toggled to drive the movement of the compression needle piece 2300, but also to ensure that in such as the compression needle piece 2300 by the foreign object stagnation affects the movement of the continued toggle 3100, so that only the compression spring 4200 will be compressed until the toggle 3100 to the end of the movement, and will not be due to the tensile force is too large to damage the relevant parts.

As shown in Figure 11, the specific working works of the luminal stitcher of the present invention are as follows: in the process of executing the operation, the working end 2000 can be rotated arbitrarily around the A-axis line according to the need, and the angle of rotation can be up to 360 degrees; after executing the relevant operation, as shown in Figure 9, toggle the toggle lever is provided with the toggle button 3103, which leads the wire rope 3300 to drive the rotating shaft sleeve 1200 to do axial movement, and the rotating shaft sleeve 1200 drives the rotating inner shaft 1300 to do axial movement, and the rotating inner shaft drives the compression needle piece 2300 to overcome the compression of the compression spring to do axial movement through the steel wire shaft, so as to lift the restriction on the suture needle 2400; after completing the relevant operation of the suture needle, the toggle lever is loosened, so as to loosen the steel wire rope 3300, and the compression needle piece 2300 resumes the initial position under the compression of the compression spring 2200. The luminal suture apparatus of the present invention makes it possible to realize 360-degree rotation of the working end to meet the needs of the surgery in the specific operation works, and at the same time, in the rotation works of the working end 2000, the wire rope does not need to be rotated, so as not to cause the wire rope 3300 to become entangled and cause the failure of the instrument, so as to ensure the safety of the surgery.

Other embodiments of the invention will readily come to mind to those skilled in the art upon consideration of the invention disclosed in the specification and embodiments. This application is intended to cover any variations, uses, or adaptations of the present invention which follow the general principles of the invention and include means of common knowledge or customary skill in the art not disclosed herein. The specification and embodiments are to be regarded as exemplary only, and the true scope and spirit of the invention is indicated by the following claims.

It should be understood that the present invention is not limited to the precise structure which has been described above and illustrated in the accompanying drawings, and that various modifications and alterations may be made without departing from its scope. The scope of the invention is limited only by the appended claims.

## Claims

1. An endoscopic suture device, comprising: a working end, a pressing-piece driving component, wherein the working end comprises a pressing-piece, the pressing-piece is used for restraining a suture needle from being separated from a track, and the pressing-piece driving component is able to release the restraint of the pressing-piece on the suture needle, **characterized in that**:
the pressing-piece driving component comprises: a rotating shaft sleeve, a rotating inner shaft, a first limiting component, a steel wire shaft, a steel wire rope; the rotating inner shaft is arranged in the rotating shaft sleeve, the first limiting component is used for limiting the rotating inner shaft so that the rotating inner shaft is not able to move axially relative to the rotating shaft sleeve but is able to rotate relative to the rotating shaft sleeve; one end of the steel wire shaft is fixedly connected to the pressing-piece, and the other end is fixedly connected to the rotating inner shaft; one end of the steel wire rope is fixedly connected to the rotating shaft sleeve.

2. The endoscopic suture device according to claim 1, **characterized in that**, at least one compression spring is further provided at the working end, a compression spring hole is provided on the pressing-piece, and the compression spring is fitted into the compression spring hole to apply compression to the pressing-piece so as to restrain the suture needle from departing from the running track; the pressing-piece is provided with a steel shaft hole for inserting one end of the steel shaft and performing a fixed connection, and the rotating inner shaft is provided with a circular hole for inserting the other end of the steel shaft and performing a fixed connection; the rotating inner shaft is provided with a connection hole for fitting into one end of the steel wire rope and fixedly connecting.

3. The endoscopic suture device as claimed in claim 2, **characterized in that** the first limiting member comprises a U-shaped annular groove and a spherical steel ball, wherein the U-shaped annular groove is provided on an outer side wall of the rotating inner shaft, and the rotating shaft sleeve is provided with a plurality of steel ball holes in a radial direction, a spherical steel ball is mounted in each steel ball hole, and the spherical steel ball has a first protruding portion protruding from the inner surface of the rotating shaft sleeve, the first protruding portion is correspondingly provided in the U-shaped annular groove, and the height of the first protruding portion is smaller than the depth of the U-shaped annular groove.

4. The endoscopic suture device as claimed in claim 2, **characterized in that** said endoscopic suture device also comprises a fixed outer tube and a second limiting member, the rotating shaft is sleeved in the fixed outer tube, and the second limiting member is used for limiting the rotating shaft sleeve so that the rotating shaft sleeve is not able to rotate relative to the fixed outer tube but is able to move linearly along the axis relative to the fixed outer tube.

5. The endoscopic suture device according to claim 4, **characterized in that** the specific structure of the second limiting member is as follows: the spherical steel ball has a second protruding portion protruding from the outer circumferential surface of the rotating shaft sleeve, and the fixed outer tube is provided with an axial track groove arranged corresponding to the second protruding portion of the spherical steel ball, wherein the height of the second protruding portion is smaller than the depth of the axial track groove;
or, the spherical steel ball is not higher than the surface of the outer side wall of the rotating shaft sleeve after being inserted into the rotating shaft sleeve, the outer side wall of the rotating shaft sleeve is provided with at least one track protrusion, the inner side wall of the fixed outer tube is correspondingly provided with at least one track groove, and the height of the track protrusion is less than the depth of the track groove.

6. The endoscopic suture device according to any one of claims 1-5, **characterized in that** the endoscopic suture device also comprises a skeleton, a shifting lever is provided on the skeleton, one end of the steel wire rope is fixed on the shifting lever; when the shifting lever is shifted, the steel wire rope is pulled to move.

7. The endoscopic suture device according to claim 6, **characterized in that** the skeleton is provided with a first pin hole and a second pin hole respectively used for inserting the first pin shaft and the second pin shaft; one end of the steel wire rope passes through a steel wire hole provided on the shifting lever and is fixed; the shifting lever is provided with a second pin shaft hole for passing through a second pin shaft, and the shifting lever can rotate by taking the second pin shaft as an axis; further comprising a steel wire rotating wheel, wherein the steel wire rotating wheel is provided with a central hole for inserting the first pin shaft; and the steel wire rope is embedded into a U-shaped groove provided on the steel wire rotating wheel.

8. The endoscopic suture device according to any one of claims 1-5, **characterized in that** the device further comprises a cushioning component, the cushioning component is disposed between the shift lever and the rotating shaft sleeve, the steel wire rope is broken at the cushioning component into two, and two ends of the cushioning component are connected to the steel wire ropes respectively so as to connect the broken steel wire ropes; the cushioning component comprises a cushioning sleeve, a cushioning compression spring, a cushioning sleeve cover, and a spring compression block.

9. The endoscopic suture device as claimed in claim 8, **characterized in that** the cushioning compression spring and the spring compression block are disposed in the cushioning sleeve, one end of the cushioning compression spring abuts the inner side of the bottom of the cushioning sleeve, and the other end abuts the spring compression block; one steel wire rope passes through the bottom through-hole of the cushioning sleeve tube, a cushioning compression spring and a spring pressing block in sequence and is then fixed; and the other steel wire rope passes through the cover of the cushioning sleeve tube and is then fixed, and the cushioning sleeve tube is fixedly connected to the cover of the cushioning sleeve tube.

10. The endoscopic suture device according to claim 9, **characterized in that** the elastic force Fcushioning compression spring of the cushioning compressing spring is less than the tensile strength of the steel wire rope and the strength of other relevant parts, and satisfies the following relationship: Fcushioning compression spring>2×Fcompression spring, wherein Fcompression spring refers to the elastic force of the compression spring at the working end.
